# EUROPÄISCHE PATENTSCHRIFT

(11) **EP 2 651 373 B1**
(45) Veröffentlichungstag und Bekanntmachung des Hinweises auf die Patenterteilung: **17.05.2017**
(21) Anmeldenummer: 11796624.2
(22) Anmeldetag: 07.12.2011
(51) Int. Cl.: A61K 8/14, A61K 8/46, A61K 8/86, A61K 8/92, A61Q 19/10

(54) **LIPOSOMENBILDENDE WÄSSRIGE BADEZUBEREITUNGEN MIT HOHEM WIRKSTOFFANTEIL UND DEREN ANWENDUNG**
LIPOSOME-FORMING AQUEOUS BATH PREPARATIONS HAVING A HIGH PROPORTION OF ACTIVE SUBSTANCE AND USE OF SAID PREPARATIONS
PRÉPARATIONS AQUEUSES POUR LE BAIN FORMANT DES LIPOSOMES PRÉSENTANT UNE FORTE TENEUR EN PRINCIPE ACTIF ET APPLICATION DESDITES PRÉPARATIONS

(30) Priorität: 14.12.2010 DE 102010054461
(43) Veröffentlichungstag der Anmeldung: 23.10.2013
(73) Patentinhaber: Merz Pharma GmbH & Co. KGaA, 60318 Frankfurt am Main (DE)
(72) Erfinder: EBINGER, Jürgen, 65510 Hünstetten 8 (DE); HOLZEM, Martina, 71083 Herrenberg (DE)
(74) Vertreter: Müller, Claudia
(86) Internationale Anmeldenummer: PCT/EP2011/006116
(87) Internationale Veröffentlichungsnummer: WO 2012/079717

(56) Entgegenhaltungen:
- WO-A2-2011/006616
- DE-A1- 10 231 736
- DE-A1- 19 922 193
- DE-A1-102005 056 538
- US-A1- 2008 095 725
- US-A1- 2010 178 329
- Eintrag in Wikipedia zu Ätherischen Ölen
- Anonymous: "RÖMPP - Lipide - Georg Thieme Verlag KG", , 1 April 2007 (2007-04-01), XP055238305, Retrieved from the Internet: URL:https://roempp.thieme.de/roempp4.0/do/ data/RD-12-01258 [retrieved on 2015-12-23]
- ANONYMOUS: 'RÖMPP - ätherische Öle - Georg Thieme Verlag KG', [Online] 01 Dezember 2007, XP055238307 Gefunden im Internet: <URL:https://roempp.thieme.de/roempp4.0/do/ data/RD-01-04781> [gefunden am 2015-12-23]

## Beschreibung

### Gegenstand der Erfindung

Die vorliegende Erfindung betrifft flüssige kosmetische, dermatologische oder medizinische liposomenbildende wässrige Badezubereitungen mit einem hohen Anteil von mehr als 8, vor allem 10 bis 27 Gew. % an Wirkstoff(en), insbesondere ätherischen Ölen, sowie einem oder mehreren Tensiden zu 20 bis 55 Gew.%, und Vesikelbildnern. Derartige Zubereitungen zum Baden bilden überraschender Weise trotz der hohen Anteile an Wirkstoff bzw. Tensid beim Einbringen in Wasser spontan liposomale Strukturen aus, welche die Wirkstoffe zusammen mit weiterhin enthaltenen Lipiden an und in die Haut transportieren. Dazu sind weder besondere Stabilisierungsmaßnahmen noch der Einsatz bestimmter Zusätze erforderlich, wenn erfindungsgemäß eine bestimmte Wahl an Lipiden, insbesondere umfassend ein oder mehrere, vor allem natürliche, Öle mit einem hohen Gehalt an Öl- und Linolsäure getroffen wird.

### Hintergrund der Erfindung

Balneologische Zusätze zur Anwendung in der Wanne oder der Dusche sind seit langem bekannt. Dabei werden wasserfreie, insbesondere Öl- haltige Produkte von wasserhaltigen Produkten unterschieden, welche je nach Zusatzstoffen unterschiedliche Konsistenzen von flüssig bis gelartig aufweisen können. Sie enthalten vor allem Tenside zur Reinigung der Haut sowie vorzugsweise lipidische Substanzen, um den durch die Anwendung von Wasser und Tensiden entfernten Lipidfilm wieder aufzubauen.

Vesikuläre Systeme haben als Wirkstoffträger sowie Hautpflegekomponenten eine breite Anwendung in Kosmetik und Pharmazie gefunden. So sind kosmetische Liposomen- bzw. Vesikel-haltige Gele und Emulsionen bekannt, wobei wegen der Stabilitätsprobleme der Vesikel gegenüber Elektrolyten, Emulgatoren (Tensiden) und Wasser die liposomalen Anteile extern als Vesikel- Dispersionen gebildet und dann in die Formulierung eingebracht werden. Ein solches Verfahren ist jedoch aufwendig. Dementsprechend wurden Mittel gefunden, liposomale Strukturen in situ bei der Anwendung zu erzeugen. So werden gemäß der DE 42 05 548 A1 bestimmte wasserdispergierbare öllösliche polyoxyethylierte Tenside mit einem HLB-Wert von 6-13 zusammen mit Vesikelbildnem wie Phospholipiden in einer Ölgrundlage beschrieben, welche erst bei Kontakt mit Wasser, z. B. Eingießen in die Badeflotte, liposomale Strukturen ausbilden. Dadurch kann eine protrahierte Wirkstofffreisetzung mit Depoteffekt erzielt werden. Der Wirkstoffanteil beträgt hier jedoch wesentlich weniger als 10 Gew.%. Ferner ist die Zubereitung im Wesentlichen nicht wasserhaltig. Um einen hohen Wirkstoffanteil (mehr als 20 Gew.%) in eine tensidische wässrige Reinigungszubereitung (1 bis 60 Gew.% irgendeines Tensids) zu inkorporieren, mit welcher dennoch in situ eine Vesikelbildung erfolgt, wird gemäß der DE 102 31 736 A9 eine Mischung aus ethoxylierten und nicht ethoxylierten Sterolen verwendet. Hier wird jedoch auf den Einsatz an Fetten oder Ölkomponenten (Lipiden) verzichtet, da der beim Reinigen eintretende Verlust der Haut an Lipiden mittels der genannten Sterolkombination ausgeglichen werden soll. Ferner muss zur Herstellung der Zubereitung eine Lösung aus Wasser und Tensiden hergestellt und diese dann auf die Schmelztemperatur der Sterole erwärmt werden. Sodann werden die Sterole darin geschmolzen, anschließend die Mischung kalt gerührt und danach ggf. weitere Zusatz-, Hilfs- und Wirkstoffe eingearbeitet.

In der DE 40 21 083 A1 werden Alkoholhaltige wasserfreie in situ Liposomenbildende Phospholipidbäder mit Ölanteil beschrieben, welche jedoch kein Tensid aufweisen.

Die DE 198 54 827 A1 betrifft vesikelbildende tensidhaltige lipid(öl)freie Reinigungszubereitungen, wobei die Vesikelbildung durch Sterole, insbesondere eine Mischung aus ethoxylierten und nichtethoxylierten Sterolen aufweist, analog der o.g. DE 102 31 736 A9, jedoch mit wenig Wirkstoffanteil und vorzugsweise umgekehrtem Sterolverhältnis.

Somit werden bisher liposomale Strukturen entweder ohne hohen Wirkstoffanteil oder ohne wesentlichen Lipidanteil / Wasser und zum Teil auch mittels aufwendiger Verfahren bereitgestellt.

### Aufgabe vorliegender Erfindung

Aufgabe vorliegender Erfindung ist es, den o.g. Nachteilen zu begegnen und eine balneologische Zubereitung zur Verfügung zu stellen, welche neben Wasser sowohl einen hohen Wirkstoffanteil, ferner einen Lipid- und Tensidanteil aufweist und dennoch in situ liposomale Strukturen entfaltet, ohne dass hierzu aufwendige Herstellungsverfahren und / oder Stabilisierungsmaßnahmen erforderlich wären.

### Lösung der Aufgabe

Diese Aufgabe wird erfindungsgemäß gelöst mit balneologischen Zubereitungen zur kosmetischen Anwendung, welche Bestandteile gemäß Anspruch 1 aufweisen. Wirkstoffe sind dabei bevorzugt in Mengen von 10 bis 25 Gew %, vor allem 12 bis 25 Gew. %, insbesondere 15 bis 20 Gew. % enthalten. Die Lipidkomponente ist insbesondere in Mengen von 2 bis 5 Gew. % enthalten. Vesikelbildende Lipide (Vesikelbildner), insbesondere Phospholipide, sind insbesondere in Mengen von 0,3 bis 5 Gew.% enthalten. Die Menge an Wasser beträgt vor allem 7 bis 60 Gew. %, vorzugsweise 10-57 Gew.%, insbesondere 13 bis 54 Gew.%.

Alle %-Angaben beziehen sich auf das (Gesamt)Gewicht der Mischung.

Die erfindungsgemäßen Zubereitungen können darüber hinaus Zusatzstoffe bis 25 Gew.%, vor allem 0,1 bis 20 Gew.% und / oder Zusatzwirkstoffe (0 bis 15 Gew.%, vor allem 0,1 bis 15 Gew.%) aufweisen. Als Zusatzstoffe sind vor allem Parfümstoffe, Konsistenzregler (Konditioniermittel), Konservierungsmittel, Stabilisatoren, Farbstoffe geeignet.

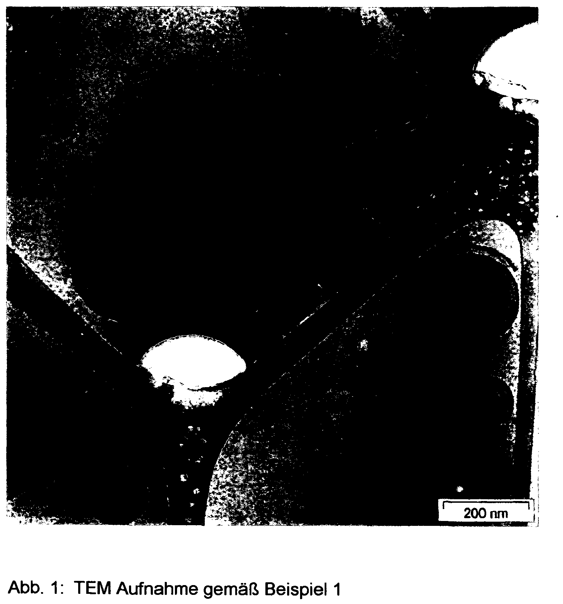

Als Zusatzwirkstoffe kommen vor allem Pflegestoffe, Feuchthaltemittel, Vitamine, oder Mischungen hiervon infrage. Insbesondere können die Zusatzwirkstoffe auch ausgewählt werden im Hinblick auf einen balneologisch insgesamt oder zusätzlich beabsichtigten Effekt, wie z.B. Erfrischung, Belebung, Anti-Stress, Muskel-Relax, Durchblutungsförderung, Abhärtungsbad oder Erkältungslinderung, Beruhigung, Linderung rheumatischer oder Kreislauf- Beschwerden oder Hautirritationen. Die erfindungsgemäßen Zubereitungen bilden trotz des hohen Wirkstoff- und Tensidanteils überraschenderweise spontan liposomale Strukturen in der wässrigen Badeflotte. Diese können mittels Transmissionselektronenmikroskopie (TEM) nach Gefrierbruch nachgewiesen werden und sind in den nachfolgenden Abb. 1 bis 2 gemäß Beispiel 1 und 2 der Anmeldung aufgezeigt.

Mit derartigen balneologischen Zubereitungen ist es somit möglich, Produkte mit sich spontan bildenden Liposomen und einem erhöhten Wirkstoffanteil neben einem Wasser- und Lipidgehalt als Reinigungs- und Pflegemittel einzusetzen. Dabei sind diese Präparate sehr gut verteilungsfähig und lösen sich bei Eintrag in Wasser vollständig klar auf. Sie verfügen ferner über eine sehr gute Stabilität. Sie können darüber hinaus ohne Einsatz spezieller und aufwendiger Apparaturen und Verfahren, wie sie üblicherweise zur Bereitstellung liposomaler Präparate erforderlich sind, auf einfachste Weise hergestellt werden. Hierbei zeigte sich überraschenderweise, dass insbesondere die Anwesenheit von Tensiden und hohe Mengen an Wirkstoff(en) keine negative Auswirkung auf das Vorhandensein bzw. die Ausbildung der Liposomen hat. Dies konnte nicht erwartet werden im Hinblick auf den bereits geschilderten Stand der Technik, worin festgestellt wurde, dass herkömmliche liposomale Strukturen durch solche stark oberflächenaktiven Tenside sowie Wasser solubilisiert werden bzw. der Wirkstoffanteil begrenzt und / oder bestimmte Herstellungsverfahren und Strukturbildner wie Sterolmischungen erforderlich sind. Mit den anmeldungsgemäßen Zubereitungen können somit mehrere Effekte möglich sein, nämlich zum einen eine optimale Vorbereitung der Haut zur Aufnahme von Substanzen/Wirkstoffen durch die Anwesenheit der Tenside, zum anderen eine verbesserte, insbesondere protrahierte und damit nachhaltige Hautwirkung aufgrund des durch die vorhandenen liposomalen und Lipid-Bestandteile bewirkten effektiven Wirkstofftransports an und auch tiefer in die Haut im Sinne eines Sofort- bzw. auch eines nachhaltigen (Depot-)Effekts. Insbesondere können vor allem auch natürliche Wirk-Substanzen (Wirkstoffe), Lipide, Vesikelbildner eingesetzt werden.

### Abbildungen

In den Abbildungen entsprechend den Fig. 1 -2 werden die liposomalen Strukturen (Vielzahl kugelförmiger Gebilde) der Zubereitungen gemäß den Beispielen 1 - 2 nach Eingießen in Wasser durch Transmissionselektronenmikroskopie nach Gefrierbruch dargestellt. Ferner ist eine Aufnahme gemäß Vergleichsbeispiel 6 sowie eine Aufnahme gemäß Vergleichsbeispiel 7 (Fig. 3, Fig. 4) beigefügt. Wie hier deutlich sichtbar, sind ohne den Zusatz anmeldungsgemäßer Lipide nahezu keine Liposomen erkennbar.

### Bevorzugte Ausführungsformen

Bevorzugt umfasst die Zubereitung 1 bis 6 Gew.% Lipide (als Lipidkomponente), vor allem flüssige Lipide, wie natürliche Öle oder ggf. Gemische flüssiger Lipide mit (halb)festen Lipiden; 25 bis 49, vor allem 30 bis 45 Gew.% Tenside, 12 bis 23 Gew.% Wirkstoffe; 0,3 bis 4 Gew. % vesikelbildendes Lipid(e); 0 bis 25, vor allem 0,1 bis 25 oder bis 15 Gew. % Zusatzstoffe und ggf. (0 bis 15, vor allem 0,5 bis 15 Gew.%) Zusatzwirkstoffe sowie als Rest, z. B. 15 bis 53 %, Wasser.

In einer weiteren Ausführungsform kann auch ein Gemisch aus einem oder mehreren anionischen, nichtionischen und amphoteren, insbesondere aus einem oder mehreren nichtionischen und amphoteren bzw. einem oder mehreren anionischen und nichtionischen Tensid(en) eingesetzt werden.

Anionische Tenside sind solche wie nachfolgend beschrieben und insbesondere ausgewählt aus C₁₀-C₁₈- Alkylsulfaten, Sarcosinaten, Isethionaten, Ethercarbonsäuren, Eiweiß-Fettsäure-Kondensaten, C₁₀₋₈Monoalkyl-phosphaten, C₁₀-₁₈ -Mono-glyceridsulfaten, Amidethersulfaten, C₁₀-₁₈Alkylsulfoacetaten, oder Mischungen hiervon. Die Alkylketten in den letztgenannten Tensiden können dabei jeweils acht bis achtzehn Kohlenstoffatome umfassen.

Ebenfalls verwendbar sind Alkylethersulfate, die sich von Fettalkoholen mit 12 bis 18 Kohlenstoffatomen ableiten, den entsprechenden, ethoxylierten, oder propoxylierten oder gemischt oxylierten Derivaten hiervon mit einem Oxylierungsgrad von 1 bis 6, und vor allem darunter die, die einen Ethoxylierungsgrad von 2 bis 6 aufweisen, wie z.B. Lauryl/Myristylethersulfat, Na-Salz (z.B. Texapon^{®} K 14 S spezial IS), Ammoniumlaurylethersulfat (Zetesol^{®} LA-2) oder Monoisopropanolammoniumlaurylethersulfat (Zetesol^{®} 2056) oder deren Mischungen wie Zetesol^{®} 100 (=MIPA-Laureth Sulfate, Laureth-4, Cocamide DEA) bzw. Alkylsulfate, z.B. Natriumlaurylsulfat (Texapon^{®} Z), Ammoniumlaurylsulfat (Texapon^{®} ALS) oder Monoisopropanolammoniumlaurylsulfat (Sulfetal^{®} CJOT 60).

Ferner geeignet sind insbesondere C₁₀₋₁₈Alkyl-Sarcosinate wie Natriumlaurylsarcosinat.

Das nichtionische Tensid ist insbesondere ausgewählt aus C₈-C₂₂Alkanolamiden, z.B. Cocamide^{®} MEA, Cocamide DEA, C₁₀-C₁₆ Alkylpolyglucosiden, z.B. Plantacare^{®}2000 UP (Decyl Glucoside); oder Sorbitan(C₈-C₂₂ )-estern (mit Glykolen, Ethylenglykolen), z.B. Tween^{®}20 oder 80 (Polysorbate 20 oder 80), C₁₀-C₁₈ - Fettsäureethoxylate mit 8-40 EO (Ethylenoxid-)-Einheiten oder Mischungen aus vorgenannten Tensiden.

Das amphotere Tensid kann insbesondere ausgewählt werden aus C₈-C₂₂-Alkyl-aminopropionaten, C₈-C₂₂Alkyl-, C₈-C₂₂Alkylamido- und Sulfo-Betainen und C₈-C₂₂-Alkylglycinaten; oder Mischungen hiervon. Insbesondere bevorzugt ist hier Cocamidopropylbetain.

Das oder die Tenside liegen vorzugsweise in einer Gesamtmenge von 24 bis 47 Gew. % vor. Insbesondere geeignet sind nichtionische Tenside oder Mischungen von diesen mit anionischen und / oder amphoteren Tensiden.

Die erfindungsgemäße Zusammensetzung enthält weiterhin wie erwähnt einen hohen Anteil an Wirkstoffen, nämlich mehr als 8, wie 8,3 bis 27 Gew.%, vor allem 10 bis 25 Gew. %, vorzugsweise 12 bis 25 Gew.%, insbesondere 14 bis 20 Gew. %. Diese Wirkstoffe sind vorzugsweise ausgewählt aus ätherischen Ölen und Extrakten aus Pflanzen. Bevorzugte ätherische Öle / Extrakte aus Pflanzen sind insbesondere Kiefernnadel, Thymian, Minze, Limetten, Orangen, Grapefruit, Mandarine, Wacholder, Baldrian, Zitronenmelisse, Eukalyptus, Thymian, Rosmarin, Lavendel, Rosenholz, Lindenblüten-, Ringelblumen-, Algen-, Aloe Vera-, Echinacea-, Efeublätterextrakt, Terpentinöl, oder Mischungen hiervon.

Dabei können je nach beabsichtigtem Wirkungseffekt, wie z.B. eine balneologische Wirkung (z.B. Erkältungslinderung, Beruhigung), Verbesserung der Hautstruktur, Erhöhung der Durchblutung, Entspannung, Aromatherapie, eine geeignete Kombination an Wirkstoffen und ggf. Zusatzwirkstoffe wie nachfolgend genannt, z. B. den o.g. Extrakten / Ölen entsprechende synthetisch hergestellte Substanzen bzw. analoge Derivate hiervon wie vor allem Campher, Menthol, Salicylate, Huminsäuren und / oder Nicotinate, zugesetzt werden.

### Nähere Erläuterung der Inhaltsstoffe

### 1. Lipide

Die Zubereitungen vorliegender Erfindung umfassen zunächst ein oder mehrere Lipide (als Lipidkomponente) in einer dem jeweiligen Produkt angepassten Menge von 1 bis 8 Gew.%. Unter Lipidkomponente gemäß vorliegender Anmeldung wird nur die Menge an Lipiden wie in dieser Kategorie beschrieben verstanden. Andere Lipidartige oder Lipidlösliche Stoffe wie sie in erfindungsgemäßen Zubereitungen enthalten sind oder sein können (wie Zusatzstoffe, Zusatzwirkstoffe, Wirkstoffe), werden von der anmeldungsgemäß bezeichneten Lipidkomponente nicht umfasst und liegen sofern vorhanden separat vor.

Die Lipide der Lipidkomponente können ausgewählt werden aus der in Anspruch 1 bezeichneten Gruppe, umfassend bestimmte natürliche oder synthetische (gesättigte, ungesättigte) Fette / Öle bzw. halbfeste Lipide (halbfeste Wachse). Hierzu gehören natürliche, partial synthetische oder synthetische Öle, Kohlenwasserstoffe wie Squalen, Squalan, insbesondere auch flüssige Paraffine, Isoparaffine, wie Dioctylcyclohexane, Isodecan, Isohexadecane. Dabei hat sich gezeigt, dass besonders gute Resultate erfindungsgemäßer Zubereitungen erhalten werden, wenn die Lipidkomponente mindestens ein flüssiges Öl, vor allem ein natürliches Öl, umfasst.

Bevorzugte Lipide sind ausgewählt aus flüssigen Lipiden wie Ölen, insbesondere natürlichen Ölen, sowie aus halbfesten Lipiden wie Natürliche flüssige Wachse, z.B. Jojobaöl oder dessen Substitut Oleyl Erucate; Jojobaöl, Shea Butter oder Illipé Butter, oder auch aus Mischungen aus flüssigen und halbfesten Lipiden. Es ist weiterhin bevorzugt, wenn die Lipidkomponente (eine oder mehrere Lipide) in flüssiger Form vorliegt.

Zu den besonders bevorzugten Lipiden (der Lipidkomponenete) gehören vor allem flüssige Lipide, insbesondere Öle, ausgewählt vor allem aus natürlichen Ölen mit einem hohen Gehalt an Ölsäure und (+) Linolsäure. In Tabelle 1 sind Vertreter aus dieser Gruppe und deren jeweilige Säureanteile angegeben:

**Tabelle 1**

| Öl / Anteil an | Ölsäure (%) | Linolsäure(%) | ΣÖl/Linolsäure |
|---|---|---|---|
| Aprikosenkernöl | 65 | 25 | 90 % |
| Avocadoöl | 62,5 | 13,5 | 76% |
| Borretschsamenöl | 16 | 38 | mind. 54% |
| Distelöl | 14-24 | 63-79 | mind. 77% |
| Erdnussöl | 48-66 | 25-43 | mind. 73% |
| Haselnussöl | 66-83 | 8-20 | mind. 74% |
| Kürbiskernöl | 21-46 | 36-60 | mind. 57% |
| Maiskeimöl | 20-42 | 39-65 | mind. 59% |
| Mandelöl | 77 | 17-20 | mind. 94% |
| Marulaöl | 70-90 | 4-9 | mind. 74% |
| Mohnöl | 30 | 60 | 90% |
| Nachtkerzenöl | 8-12 | 70-80 | mind. 78% |
| Olivenöl | 70 | 8-12 | mind. 78% |
| Palmöl | 40 | 10 | 50% |
| Pinienkernöl | 36-39 | 47-51 | mind. 83% |
| Reiskeimöl | 40-50 | 29-42 | mind. 69% |
| Sanddornöl | 50 | 11 | 61% |
| Sesamöl | 42 | 40-50 | mind. 80% |
| Sojaöl | 23 | 51-53 | mind. 74 % |
| Sonnenblumenöl | 13-40 | 48-74 | mind. 61 % |
| Traubenkernöl | 15-25 | 63-72 | mind. 78% |
| Walnussöl | 14-30 | 47-83 | mind. 61% |
| Weizenkeimöl | 20 | 52 | 72% |

(Angaben Säuregehalt: siehe z. B. DAZ, 150. Jahrgang, 02.09.2010, Nr. 35, Seite 3925-3934).

Vorzugsweise beträgt der Anteil an Ölsäure + Linolsäure mindestens 50%, insbesondere mehr als 60% und ganz besonders bevorzugt mindestens 75 %.

Es ist ferner bevorzugt, wenn in einer Lipidmischung (der Lipidkomponente) mindestens eine Substanz mit einem Gesamtanteil von Ölsäure + Linolsäure von wenigstens 75 %, bevorzugt wenigstens 80% vorhanden ist.

Für anmeldungsgemäße Zubereitungen geeignete Öle der oben beschriebenen Art sind ausgewählt aus Olivenöl, Sonnenblumen-, Sojaöl, Aprikosenkern-, Traubenkernöl, Haselnussöl, Mohnöl-, Erdnuß-, Mandel-, Weizenkeim-, Sesam-Distelöl, Avocadoöl, Borretschsamenöl, Sanddornöl, Maiskeimöl, Walnussöl, Nachtkerzenöl, Palmöl, Pinienkernöl, Reiskeimöl, Kürbiskernöl, Marulaöl, oder Mischungen dieser Öle oder Mischungen mit halbfesten Lipiden.

Ganz besonders bevorzugte flüssige Lipide sind Pflanzenöle, ausgewählt aus Mandelöl, Avocadoöl, Marulaöl, Aprikosenkernöl, Haselnussöl, Sanddornöl, Sojaöl oder Mischungen hiervon.

Die Lipide der Lipidkomponente sind in erfindungsgemäßen Zubereitungen vorzugsweise in einer Gesamtmenge von 2 bis 7 Gew.% oder auch von 1,5 bis 6 Gew.%, vor allem 2 bis 4,5 Gew.%, enthalten.

In einer besonderen Ausführungsform können, jeweils einzeln oder auch als Mischungen, natürliche Öle oder Mischungen hiervon eingesetzt werden.

### 2. Tenside

Die Zubereitungen umfassen weiterhin ein oder mehrere (oberflächenaktive) Tenside. Hierfür geeignet sind nichtionische, anionische, und amphotere Tenside sowie Mischungen hiervon. Je nach Beschaffenheit handelt es sich um Tenside (Dispergiermittel) mit O/W- Eigenschaften und solche mit W/O- Eigenschaften.

Als nicht ionische Tensidkomponenten werden vorzugsweise höherpolyoxyethylierte / und / oder -propoxylierte- C₈₋₂₂-Fettalkohole (vor allem Alkyl-C₁₀-C₁₈-Fettalkoholalkoxylate) oder derartige ethoxylierte und / oder propoxylierte C₁₂₋₂₂₋Fettsäuren mit Ethoxylierungs- oder Propoxylierungsgraden von 8-25 oder Mischungen hiervon eingesetzt wie: Polyoxypropylenstearylether oder Polyoxyethylensorbitanmonooleat, Polyoxyethylensorbitanmonostearat - oder -palmitat, Polyoxyethylenstearylether, Polyoxyethylenoleyether, Polyoxyethylenoxypropylenmonostearat, Polyoxyethylenfettalkohol-ether, Polyoxyethylenmonostearat. Insbesondere bevorzugt sind hier ethoxylierte C₁₂-₂₂₋Fettsäuren oder ethoxylierte C₈₋₂₂₋Fettalkohole mit Ethoxylierungsgraden von 10-20.

Weiterhin geeignet sind auch nicht ionische Glykoside wie Zuckerester oder Zuckerether von C₁₂₋₂₂₋Fettsäuren oder C₈₋₂₂₋Fettalkoholen. Dazu zählen insbesondere Zuckerester von C₁₂-C₁₈ gesättigten, ungesättigten, partial gesättigten Fettsäuren; polyoxyethylierte und / oder polyoxypropylierte Zuckerester von C₁₂-C₁₈Fettsäuren, wobei der EO bzw. PO Grad 8-25 beträgt. Zu den Zuckerethern gehören solche von C₈-C₂₀ gesättigten, ungesättigten, partial gesättigten Fettalkoholen; oder von polyoxyethylierten und / oder polyoxypropylierten Zuckerethern von diesen C₈-C₂₀- Fettalkoholen. Der EO- bzw. PO Grad liegt zwischen 8 und 25. Als Zucker sind insbesondere Glukose, Methylglucose, Saccharose geeignet und als Säuren oder Alkohole die C₁₆-C₁₈ Fettsäuren / Alkohole. Bevorzugt sind ethoxylierte Produkte. Die Säure- bzw. Alkoholkomponenten leiten sich vor allem von aliphatischen Carbonsäuren / Alkoho-len ab. Hierzu gehören insbesondere Caprylsäure, Caprinsäure, Laurinsäure, Myristinsäure, Palmitinsäure, Stearinsäure, Ölsäure und auch Mischungen hiervon bzw. derartige Fettalkohole (Oleylalkohol, Laurylakohol Stearylalkohol).

In einer weiteren besonders bevorzugten Ausführungsform werden die Tenside ausgewählt aus anionischen Verbindungen wie z. B. C₈₋₂₂₋Alkylsulfaten bzw. C₈₋₂₂ - Alkylethersulfaten bzw. deren Alkali- insbesondere Natriumsalzen oder ethoxylierten Derivaten wie beschrieben wie Natriumlaurylsulfat, Natriumlaurylethersulfat, Natriumlaureth-/ Myrethsulfat oder Mischungen hiervon.

Als amphotere Tensidkomponenten eignen sich vor allem Betaine, insbesondere Fettsäureamidoalkylbetaine wie Cocosfettsäureamidopropylbetain.

Die Tensidkomponenten sind in einer der Darreichungsform angepassten (Gesamt-) Menge wie nachfolgend beschrieben vorhanden. Die Gesamtmenge an Tensiden kann bei den erfindungsgemäßen fließfähigen (flüssigen) Produkten vor allem 28 bis 45 Gew.% betragen.

Besonders bevorzugte Tensid- Komponenten werden ausgewählt aus höher polyethoxylierten oder niedrigpolypropoxylierten bzw. auch gemischt oxylierten C₁₂₋₁₈-Fettalkoholen mit einem Ethoxylierungsgrad / Propoxylierungsgrad (EO/PO-Grad) von 16 bis 28, insbesondere mit einem EO- Grad von 18 bis 24 wie vor allem derartige Macrogol- Komponenten wie Tween (20= Polyoxyethylenglykol mit 20 EO Einheiten, insbesondere in Kombination mit anionischen Mitteln wie C₈₋₂₂ Alkylsulfaten, C₈₋₂₂ - Alkylethersulfaten und / oder mit amphoteren C₁₂ -C₁₈- Fettsäureamidoalkylbetainen wie Cocosfettsäureamidopropylbetain.

### 3. Liposomale Komponente (Vesikelbildendes Lipid= Vesikelbildner)

Die erfindungsgemäßen Zubereitungen weisen wie erwähnt Vesikel- (=Liposomen-) bildende Substanzen auf. Dazu gehören vor allem Phospholipide, wie Lecithin (z.B. Ei- oder Soja-Lecithin wie z. B. kommerziell erhältliche Produkte wie Phosal 50 SA, Phosal 40 MD u. ä.), oder Phosphatidylcholin, oder -diethanolamin, Phosphatidylserin, Phospatidylinosit, z. B. aus Soja, Raps, Baumwollesamen, Ei, sowie deren Mischungen. Weitere geeignete Komponenten zur Bildung von Vesikeln (liposomalen Strukturen) sind Sphingolipide (z.B. Ceramide, Cerebroside, Sphingosin, Sphingomyelin). Derartige Substanzen sind kommerziell erhältlich.

Die Menge an vesikelbildendem Lipid (liposomaler Komponente) beträgt 0,1 bis 5 Gew. %, bevorzugt 0,3 bis 3 Gew.%. Besonderes bevorzugt sind Phospholipde, und hierunter Lecithine wie vor allem Sojalecithin, Eilecithin, oder Mischungen hiervon.

Wie erläutert, umfasst die liposomale Komponente (vesikelbildendes Lipid) gemäß vorliegender Anmeldung die hierfür beschriebene Komponente (Substanzen).

### 4. Wirkstoffe

Die erfindungsgemäßen Zubereitungen enthalten einen wie beschrieben hohen Anteil von 8, bzw. 10 und mehr Gew.% (z. B. 15 bis 19,3 Gew.%) an Wirkstoffen, welche insbesondere ausgewählt sind aus ätherischen Ölen, Pflanzenextrakten, mit den jeweiligen dem Fachmann bekannten Wirkungen wie anregend, beruhigend, heilend, antikoagulierend, erfrischend, belebend, keimreduzierend, oder Mischungen hiervon.

Zu den ätherischen Ölen / Extrakten gehören für kosmetische / dermatologische oder ggf. medizinische Badezusätze gebräuchliche ätherische Öle / Extrakte, vorzugsweise ausgewählt aus Khakiblättern, Mango, Feigen, Lavendelöl, Zedernholz, Lotusblüten, Kamillenblüten, Ylang Ylang, Ginkgo, Kiefernnadel, Zypresse, Orangen, Rosenholz, Pflaumen-, Birken- Blätterextrakt, Aloe-Vera-Extrakt, Ringelblumen,Hibiskus-, Klettenwurzel- Hamamelis-, Wassernabelkraut-, Algen-, Quitten-, Wasserlilien-, Zimtextrakt, Extrakte / Öle aus Thymian, Minze, Limetten, Orangen, Grapefruit, Mandarine, Wacholder, Baldrian, Zitronenmelisse, Eukalyptus, Thymian, Palmarosa, Zimt, Rosmarin, Rosenholz, Lemongras, Wildrose, Fichtennadel, Kiefernnadel, Ingwer-, Johannisbeer-, Lindenblüten-, Ringelblumen-, Magnolien-, Ananas-, Guave-, Echinacea-, Efeublätterextrakt, Heidelbeer-, Kaki-, Melonen- Extrakt oder Mischungen hiervon. Diese Öle werden auf bekannte Weise wie durch Wasserdampfdestillation hergestellt. Hierdurch werden z.B. ätherische Öle aus den genannten Pflanzen erhalten, welche besonders bevorzugt sind. Die Extrakte können z.B. durch Lösungsmittelextraktion (mit Alkoholen, Triglyceriden oder Kohlenwasserstoffen, Wasser, Gemischen hiervon) erhalten und als solche dann eingesetzt werden.

Analoge synthetisch hergestellte Substanzen sind beispielsweise Terpene und Terpenoide wie Campher, Menthol, Cineol, Nicotinat, Salicylate wie nachfolgend beschrieben unter 'Zusatzwirkstoffe', oder Mischungen hiervon.

Ebenso geeignet sind Huminsäuren, die auf bekannte Weise aus Torfmooren gewonnen werden können.

Durch Inkorporierung eines oder mehrerer der oben genannten Wirkstoffe, insbesondere ätherischer Öle / Extrakte können z.B. Indikationsbadezusammensetzungen je nach gewünschtem Zweck hergestellt werden, wobei ein effektiver Transport der wirksamen Komponenten erfolgen kann. So kann z.B. ein Abhärtungsbad mit Eukalyptusöl, Minzöl und Echinacea-Extrakt; ein Anregungs-Bad mit Rosmarinöl und Limettenöl oder ein Muskel-Relax-Bad mit Ingwer-Extrakt, Methylnicotinat, Hydroxyethylsalicylat und Wacholderöl oder ein Erkältungsbad mit Eukalyptus-, Kiefernnadel- und Thymianöl sowie Campher hergestellt werden.

### 5. Zusatzstoffe

Die erfindungsgemäße Zubereitung kann darüber hinaus Zusatzstoffe enthalten. Hierzu gehören Farbstoffe, Konsistenzregler (Konditioniermittel), Konservierungsstoffe, Parfümstoffe, Stabilisatoren. Diese können z. B. in folgenden Mengen vorliegen: Konditioniermittel z.B. 0,01 bis 10 %; Stabilisator(en), z.B. 0,1 bis 10 %; Konservierungsmittel(n), z.B. 0,01 bis 5 %; Farbstoffe z.B. 0,005 bis 3 %; Parfümstoffe, z.B. 0,01 bis 5 %.

Als Stabilisatoren können z.B. Komplexbildner wie Trilon^{®}BD (EDTA, Na-Salz), Mittel zur pH- Einstellung, z.B. Zitronensäure, Milchsäure, Lösungsmittel wie Propylenglykol oder Alkohole, z. B. Ethanol, Glycerol, oder auch anorganische Salze, z. B. Natriumchlorid oder Mischungen hiervon enthalten sein.

Farbstoffe sind beispielsweise für den genannten Zweck geeignete zugelassene insbesondere wasserlösliche Farbstoffe, wie z.B. Cochenillerot, Patentblau und Chinolingelb oder natürliche wasserlösliche Farbstoffe wie Rote Beete- Extrakt, oder Chlorophyll. Diese Substanzen sind dem Fachmann bekannt und können je nach beabsichtigtem Zweck (wie Meeresmilieu, grünes Erfrischungsbad, gelbes Fußbad) entsprechend ausgewählt werden. Als Konsistenzregler (Konditioniermittel), welche ggf. auch verdickend / gelbildend wirken können, gehören vor allem mehrwertige Alkohole, wie Propylenglykol, Ethylenglykol, Glycerol. Ferner gehören hierzu ggf. ethoxylierte C₁₂₋₂₂ - Fettsäurepartialester / Ether von mehrwertigen C₂₋₆- Alkoholen wie Diglyceride z. B. Glyceroldistearat; Polyol- C₁₂₋₂₂ - Fettsäureester wie Propylenglykol - Dicaprylate / -dicaprate, Cetearylalkohol, Glycerolstearat, Isopropylmyristat, Isopropylpalmitat, mittelkettige Triglyceride, Propylen-glycolester von mittelkettigen Fettsäuren, 2-Octyldodecanol, Laurinsäurehexylester, Isooctylstearat, Capryl / Caprinsäuretriglycerid, Cetylpalmitat, oder Gemische wie Hexyldecanol und Hexyldecyl Laurat und ähnliche für solche Zwecke bekannte Substanzen wie z. B. Proteine wie Pflanzenproteine oder Proteine tierischen Ursprungs z. B. mit einer mittleren Molekülmasse von je kleiner / gleich 30000 D oder Mischungen hiervon. Letztere Pflanzenproteine oder Proteine tierischen Ursprungs oder Mischungen hiervon (MW je ≤ 30000 D) sind anmeldungsgemäß jedoch im Wesentlichen nicht erforderlich und werden daher vorzugsweise nicht eingesetzt.

Als Parfümstoffe können z. B. die vorstehend genannten ätherischen Wirkstoffe (Öle, Extrakte) oder synthetische Mittel, wie z. B. Linalool, Geraniol oder Rosenöl bzw. dem Fachmann hierfür bekannte Mittel eingesetzt werden.

Zu geeigneten Konservierungsstoffen gehören Benzoesäure bzw. deren Salze, Propionsäure bzw. deren Salze, Salicylsäure bzw. deren Salze, Sorbinsäure bzw. deren Salze, 1,3-Butandiol, Benzylalkohol, Phenylethanol, 4-Hydroxybenzoesäure-methyl,-ethyl-, propyl-, -butylester. Die Art und Menge der Zusatzstoffe richtet sich nach der gewünschten Anwendungsform und dem gewünschten Zweck. Es ist bevorzugt, wenn höchstens 25 %, bevorzugt 0.1 bis 20 Gew%, vor allem auch 0,1 bis 15 oder 1 bis 10 Gew. %, insbesondere 3 bis 15 Gew.% an Zusatzstoffen vorhanden sind. Besonders bevorzugt werden Stabilisatoren, Farbstoffe, Parfümstoffe, Konsistenzregler, ggf. Konservierungsmittel oder Mischungen hiervon gewählt.

### 5. Zusatzwirkstoffe

Zusatzwirkstoffe können ausgewählt werden aus z. B. aus Pflegestoffen, Durchblutungsfördernden Stoffen, hygienischen / dermatologisch-medizinischen Wirkstoffen, Mineralstoffen, Vitaminen oder Mischungen hiervon.

Pflegestoffe sind z. B. essentielle ungesättigte Fettsäuren und deren Ester, z.B. Linol- oder Linolensäure, Glyceryl- Linoleate, Glyceryl- Linolenate. Eine weitere Gruppe von Pflegestoffen sind Feuchthaltemittel wie Glycerin, Propylenglykol oder Polyethylenglykole, Polypropylenglykol, Butylenglykol, Sorbitol oder Polymere, z.B. Polyquaternium-Typen, Collagen oder dessen Hydrolysate, Aminosäuren.

Zu den Durchblutungsfördernden Stoffen zählen (ggf. neben o.g. ätherischen Ölen /Extrakte) Hydroxyethylsalicylat, die oben genannten Terpene und Terpenoide, insbesondere Cineol, Menthol und Campher, Nikotinsäurederivate wie Methyl- oder Tocopherylnikotinat, Alpha- und Betahydroxysäuren und deren Derivate, z. B. Glykol-, Äpfel-, Zitronen-, Wein-, Milchsäure, Salicylsäure, Isopropylbenzylsalicylate, C12-13 Alkyl Lactate (Cosmacol^{®} ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D- Panthenol, Panthenyltriacetat oder auch Polyphenole, Flavonoide, z. B. Rutin, Ferulasäure und deren Ester oder Isoflavone wie Soja-Iso-flavone oder Coenzym Q 10.

Als hygienische / dermatologisch-medizinische Wirkstoffe eignen sich z. B. Benzalkoniumchlorid, Chlorhexidin, Dexpanthenol, Allantoin, Bisabolol, Mineralstoffe und Spurenelemente wie Kupfer, Zink, bzw. deren Derivate wie Zincidone^{®} (Zink PCA-Pyrrolidoncarbonsäure), Zinkglukonat oder Kupfergluconat.

Eine andere Gruppe geeigneter Zusatzwirkstoffe stellen Huminsäuren wie oben beschrieben oder auch Vitamine bzw. Derivate hiervon dar. Hier kommen eher wasserlösliche Substanzen in Betracht. Zu den wasserlöslichen Vertretern zählen insbesondere: Vitamin C bzw. geeignete Derivate hiervon wie Calcium-Ascorbat / Natrium- Ascorbat, Kalium- Ascorbat, ferner die Vitamine der B- Gruppe wie B1 (Thiamin, z. B. Thiaminnitrat), B2 (Riboflavin wie Riboflavin-5-Phosphat-Natrium ), B3 (Niacin, Nicotinsäure und Derivate hiervon wie Niacinamid), B5 (Pantothensäure) wie Calciumpantothenat oder Provitamin B5; B6 (Pyridoxin oder Pyridoxinhydrochlorid- oder -phosphat), B7 (Vitamin H, Biotin), B9 (Folsäure), B12 (Cobalamin) oder auch Carotinoide wie Beta / Alpha-Carotin, Beta-Cryptoxanthin, Lutein, Zeaxanthin, Lycopin, z. B. in entsprechenden käuflich erhältlichen Kaltwasser löslichen (CWS)- (=cold-water-soluble)-Zubereitungen.

Weitere geeignete Vitamine sind auch Vitamin E (Tocopherol und Derivate hiervon wie z. B. Tocopherolacetat), ferner Vitamin A (Retinol) und Derivate wie Retinylpalmitat, Retinylacetat, Retinyl-proprionat.

Eine weitere geeignete Vitamin- Kombination in erfindungsgemäßen Zubereitungen umfasst Vitamin A, insbesondere das -Palmitat, Vitamin- E- Acetat, Beta-Carotin, Folsäure und / oder Vitamin B5.

Auch können Vitamin C, z. B. Calcium- Ascorbat zusammen mit Niacinamid, Provitamin B5 oder Pantothensäure kombiniert werden.

Bevorzugte Zusatzwirkstoffe sind ausgewählt aus Campher, Menthol, Salicylaten, Nicotinaten, Huminsäuren oder Mischungen hiervon oder mit Vitaminen.

### Darreichungsformen

Die Zubereitungen werden insbesondere einteilig, vor allem einphasig in flüssiger Form, dargereicht und umfassen die gesamte Mischung, aus der der Anwender eine geeignete Menge entnehmen kann, wie z. B. durch Ausgießen. Die Verpackung / Umverpackung kann dabei Hinweise für die jeweilige Anwendungsmenge aufweisen. Ggf. kann es von Vorteil sein, eine kleinere Einzelverpackung mit einer geeigneten vordosierten Menge an Zubereitung bereitzustellen wie z. B. kleinere Einzelgebinde für Reisen, Einmalanwendung. Diese können dann als Einmaldosierung leicht angewendet werden.

Für die flüssigen Produkte wie z. B. solche für ein Vollbad, ein Armbad, ein Fußbad, werden Zubereitungen hergestellt, welche 1,5 bis 7 Gew. % Lipid(e) (Lipidkomponente), vor allem 2 bis 5 Gew.% Lipid(e), 25 bis 50, insbesondere 32-47 Gew. % Tensid(e), 11 bis 23 Gew. %, vor allem 14 bis 20 Gew. % Wirkstoff(e) wie Indikationsbezogene Wirkstoffe, 0,5 bis 3 Gew.% Liposomenbildner wie vor allem Phospholipide; 0,1 bis 15 Gew. % geeignete Zusatzstoffe wie vor allem Konditioniermittel wie Glycerin, NaCl, Propylenglyol, und / oder Parfüm-, Farbstoffe, Konservierungsmittel, und 0 bis 8 Gew.%, vor allem 0,1 bis 8 Gew. % Zusatzwirkstoffe wie oben genannt, sowie als Rest, vor allem 7 bis 60 Gew. % Wasser, aufweisen. Derartige Zubereitungen sind fließfähig bis flüssig, vorzugsweise niedrig viskos.

Als besonders geeignete Zubereitungen erwiesen sich hier solche, die 2 bis 4 Gew. % Lipid(e) (z. B. natürliche Öle wie beschrieben), 25 bis 40 Gew.% Tensid(e), vor allem anionische und nicht ionische Tenside; 1 bis 3 Gew. % Phospholipid(e); 14 bis 20 Gew.% Wirkstoffe, 0,1 bis 10 Gew. % Zusatzstoffe, 0, bzw. 0,3 bis 8 Gew.% Zusatzwirkstoffe und als Rest (z. B. 10 bis 57 Gew.%) Wasser enthalten.

### Herstellung

Die erfindungsgemäßen Zubereitungen werden nach üblichen Verfahren in üblichen Apparaturen (Edelstahlkessel mit Rührwerk / Mischer) auf einfache Weise hergestellt, in denen die einzelnen Komponenten nacheinander zusammengegeben, gemischt und nachfolgend konfektioniert werden. Dabei werden vorzugsweise die Tenside vorgelegt und einzelne Zusatzstoffe sofern vorhanden, die Wirkstoffe, Lipide (Lipidkomponente), vesikelbildende Lipide und sodann Wasser und ggf. Farb-Konservierungsmittel, insbesondere bei Raumtemperatur hinzugegeben. Je nach Konfektionierungsart, z.B. Erkältungsbad, Erfrischungsbad, Entspannungsbad, Fußbad, können dabei solche Zusatzstoffe, Wirkstoffe zugesetzt werden, die hierfür üblicherweise bzw. zwecksgerichtet wie oben beschrieben verwendet werden. Zweckmäßiger Weise werden die fertigen Produkte dicht verschlossen.

### Anwendung

Die beschriebenen Zubereitungen können in der Wanne für Teil- oder Vollbäder für kosmetische, dermatologische oder medizinische Zwecke eingesetzt werden. Dabei wird vom Anwender die geeignete Menge eingesetzt, im Allgemeinen entsprechend einer auf der die Zubereitung enthaltenden Packung angegebenen Menge. Je nach Anwendungszweck wie Erfrischungsbad, Thermalbad, Entspannungsbad, Muskellockerung, Fußbad, medizinisches Bad (Erkältung, Rheuma) können geeignete (Zusatz)-Wirkstoffe enthalten sein. Neben der dadurch bedingten Wirkung erfolgt aufgrund der besonderen Kombination der Grundstoffe eine verbesserte Hautwirkung durch den liposomal ermöglichten Wirkstoff- und verbesserten Lipidtransport.

Die Dauer der Anwendung richtet sich nach dem jeweiligen z. B. Sekundärziel, wie z. B. 10 bis 30 Minuten Wannebad / Fußbad. In der Wanne sollte die Temperatur des Wassers zwischen 33° C und 42 ° C betragen, ebenso beim Fußbad. Die Zubereitungen sind so konzipiert, dass sie sich klar auflösen, so dass nach Beendigung der Anwendung keine besonderen Maßnahmen zum Entfernen eventueller Rückstände zu ergreifen sind. Nachspülen mit Wasser und / oder Abwischen, sofern gewünscht, kann jedoch erfolgen.

### Beispiele

Die Erfindung wird anhand der nachfolgenden Beispiele näher erläutert. Die angegebenen Mengen bedeuten darin Gew.%, sofern nichts anderes angegeben ist.

**Beispiel 1 (Abbildung Fig. 1) Abhärtungsbad**

| INCI-Bezeichnung / Handelsname | Gew. % |
|---|---|
| Polysorbate 20 | 30,00 |
| Sodium Myreth Sulfate | 10,00 |
| Eucalyptusöl | 10,00 |
| Kiefernnadelöl | 5,00 |
| Rosmarinöl | 3,00 |
| Thymianöl | 1,00 |
| Campher | 3,00 |
| Parfüm | 2,00 |
| Mandelöl | 3,00 |
| Sojalecithin (Phosal 50 SA) | 2,00 |
| Propylenglykol | 6,00 |
| Farbstoff Patentblau | 0,05 |
| Farbstoff Chinolingelb | 0,20 |
| Benzylalkohol | 2,00 |
| Wasser | 22,75 |
| Summe | 100,00 |

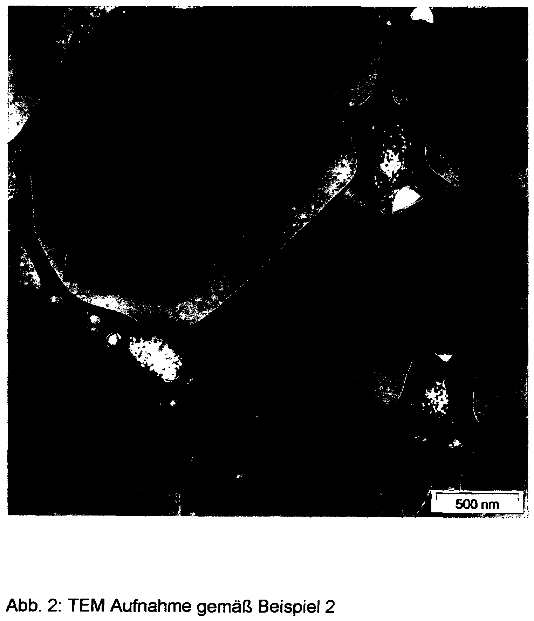

**Beispiel 2 (Abbildung Fig.2) Entspannungsbad**

| INCI-Bezeichnung / Handelsname | Gew. % |
|---|---|
| Caprylyl/ Capryl Glucoside | 40,00 |
| Cocoamidopropyl Betaine | 10,00 |
| Citronellöl | 10,00 |
| Zitronenmelissenextrakt | 3,00 |
| Mandarinenöl | 1,00 |
| Sojaöl | 4,00 |
| Orangenöl | 5,00 |
| Sojalecithin (Phosal 50 SA) | 2,00 |
| Cocoglyceride, Glyceryl Oleate | 4,00 |
| Caprylic/Capric Triglyceride | 2,00 |
| Farbstoff Cochenillerot | 0,10 |
| Farbstoff Chinolingelb | 0,10 |
| Benzylalkohol | 0,70 |
| Citric Acid | 0,10 |
| Parfüm | 1,00 |
| Wasser | 17,00 |
| Summe | 100,00 |

**Beispiel 3 Erfrischungsbad**

| INCI-Bezeichnung / Handelsname | Gew. % |
|---|---|
| Decyl Glucoside | 5,00 |
| Cocoamidopropyl Betaine | 4,00 |
| Sodium Laureth Sulfate | 15,00 |
| Aloe Vera Extrakt | 5,50 |
| Wasserlilien Extrakt | 2,00 |
| Orangenöl | 0,50 |
| Erdnussöl | 1,00 |
| Sojaöl | 2,00 |
| Sojalecithin (Phosal 50 SA) | 4,00 |
| Wasser | 45,80 |
| Parfüm | 2,50 |
| Glycerol | 10,00 |
| Coco Glucoside, Glyceryl Oleate | 1,00 |
| Farbstoff Chinolingelb | 0,08 |
| Farbstoff Grün | 0,02 |
| Benzylalkohol | 0,70 |
| Panthenol | 0,40 |
| Tocopherolacetat | 0,50 |
| Summe | 100,00 |

**Beispiel 4 Durchblutungsbad**

| INCI-Bezeichnung / Handelsname | Gew. % |
|---|---|
| Sodium Lauroyl Sarcosinate | 15,00 |
| Cocamidopropyl Betaine | 10,00 |
| Sodium Laureth Sulfate | 8,00 |
| Lavendelöl | 1,00 |
| Rosenholzöl | 0,50 |
| Palmarosaöl | 0,60 |
| Rosmarinöl | 5,00 |
| Birkenblätterextrakt | 1,00 |
| Traubenkernöl | 1,00 |
| Avocadoöl | 1,70 |
| Phosphatidylcholin | 1,50 |
| Wasser | 46,90 |
| Parfüm | 2,00 |
| Glycerol | 1,00 |
| Caprylic/Capric Triglycerid | 1,00 |
| Hydroxyethylsalicylat | 2,50 |
| Farbstoff Cochenillerot | 0,20 |
| Phenoxyethanol | 0,80 |
| Lactic Acid | 0,30 |
| Summe | 100,00 |

**Beispiel 5 Muskel- Relax- Bad**

| INCI-Bezeichnung / Handelsname | Gew. % |
|---|---|
| Polysorbat 20 | 15,00 |
| Sodium Laureth Sulfate | 10,00 |
| Cocoamidoproypl Betaine | 5,00 |
| Campher | 1,00 |
| Fichtennadelöl | 8,00 |
| Eukalyptusöl | 5,00 |
| Orangenöl | 9,00 |
| Pfefferminzöl | 3,00 |
| Sojaöl | 2,50 |
| Wildrosenöl | 1,00 |
| Phosphatidylcholin | 1,80 |
| Wasser | 34,60 |
| Hydroxyethylsalicylat | 1,40 |
| Propylenglykol | 2,00 |
| Farbstoff Patentblau | 0,20 |
| Sodium Benzoate | 0,50 |
| Summe | 100,00 |

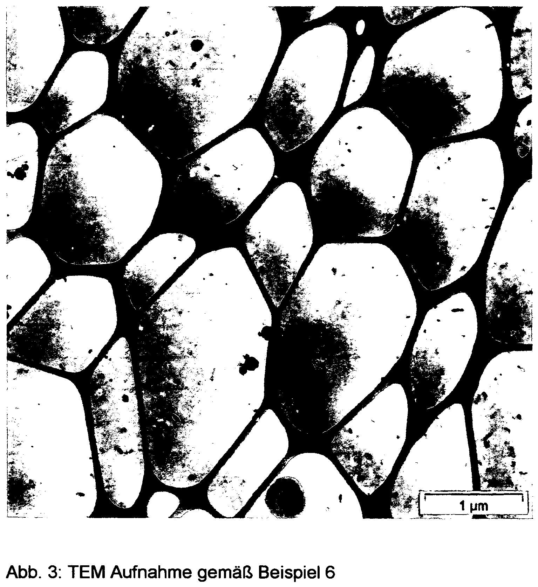

**Vergleichsbeispiel 6 (Abbildung Fig.3) Abhärtungsbad (Bsp.1) ohne Lipide**

| INCI-Bezeichnung / Handelsname | Gew. % |
|---|---|
| Polysorbate 20 | 30,00 |
| Sodium Myreth Sulfate | 10,00 |
| Eucalyptusöl | 10,00 |
| Kiefernnadelöl | 5,00 |
| Rosmarinöl | 3,00 |
| Thymianöl | 1,00 |
| Campher | 3,00 |
| Parfüm | 2,00 |
| Sojalecithin (Phosal 50 SA) | 2,00 |
| Propylenglykol | 6,00 |
| Farbstoff Patentblau, | 0,05 |
| Farbstoff Chinolingelb | 0,20 |
| Benzylalkohol | 2,00 |
| Wasser | 25,75 |
| Summe | 100,00 |

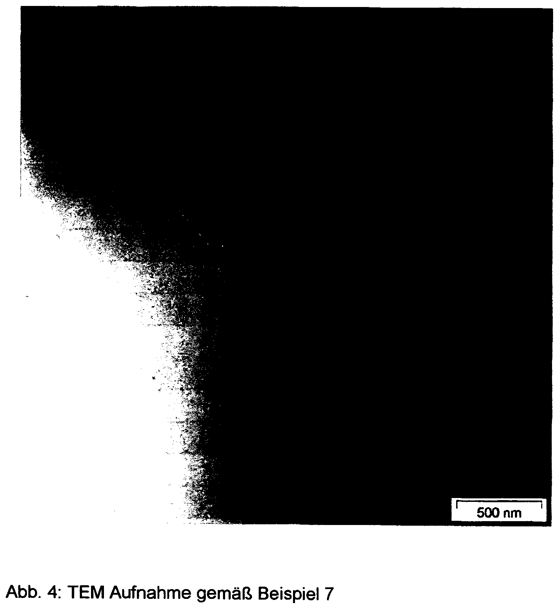

**Vergleichsbeispiel 7 (Abb. 4) Abhärtungsbad (Bsp.1) ohne Lipide, liposomale Komponente**

| INCI-Bezeichnung / Handelsname | Gew. % |
|---|---|
| Polysorbate 20 | 30,00 |
| Sodium Myreth Sulfate | 10,00 |
| Eucalyptusöl | 10,00 |
| Kiefernnadelöl | 5,00 |
| Rosmarinöl | 3,00 |
| Thymianöl | 1,00 |
| Campher | 3,00 |
| Parfüm | 2,00 |
| Propylenglykol | 6,00 |
| Farbstoff Patentblau, | 0,05 |
| Farbstoff Chinolingelb | 0,20 |
| Benzylalkohol | 2,00 |
| Wasser | 27,75 |
| Summe | 100,00 |

### Beispiel 8

### Nachweis der liposomalen Strukturen in den erfindungsgemäßen Zubereitungen mittels TEM

Zum Nachweis liposomaler Strukturen wurden nach bekannter Methode Transmissionselektronenmikroskopie - Aufnahmen nach Gefrierbruch (TEM- Aufnahmen) wie folgt aufgenommen: die jeweilige Ölbadprobe gemäß den Beispielen 1, 2, 6 bzw. 7 wurde in destilliertes Wasser analog den realen Badeflotteverhältnissen eingetragen. Die erhaltenen Mischungen wurden auf ein geeignetes Präparationsnetz aufgebracht und bei ca. 100 K gefroren. Anschließend wurde die Probe elektronenmikroskopisch analysiert. Das Ergebnis ist aus den Abb. 1 bis 4 ersichtlich. Die erfindungsgemäßen Zubereitungen entsprechend den Beispielen 1 und 2 zeigen deutlich die liposomalen Strukturen (Vielzahl kleiner kugelförmiger Gebilde, Fig. 1, 2), während mit der Zubereitung gemäß Beispiel 6 bzw. 7 ohne den Zusatz anmeldungsgemäßer Lipide nahezu keine Liposomen (Fig. 3, 4) gebildet werden.

**Liste der TEM-Aufnahmen**

| | |
|---|---|
| Beispiel 1 | = Abb. Nr. 1 (Erf.) |
| Beispiel 2 | = Abb. Nr. 2 (Erf.) |
| Beispiel 6 | = Abb. Nr. 3 (Vgl.) |
| Beispiel 7 | = Abb. Nr. 4 (Vgl.) |

## Patentansprüche

1. Liposomenbildende Badezubereitung, umfassend:
Wasser;
1 bis 8 Gew. % eines oder mehrerer Lipide als Lipidkomponente, ausgewählt aus flüssigen Ölen, ausgewählt aus Aprikosenkernöl, Avocadoöl, Borretschsamenöl, Distelöl, Erdnussöl, Haselnussöl, Kürbiskernöl, Maiskeimöl, Mandelöl, Marulaöl, Mohnöl, Nachtkerzenöl, Olivenöl, Palmöl, Pinienkernöl, Reiskeimöl, Sanddornöl, Sesamöl,Sojaöl,Sonnenblumenöl,Traubenkernöl,Walnussöl,Weizenkeimöl, aus halbfesten Lipiden der Gruppe, umfassend Jojobaöl oder dessen Substitut Oleyl Erucate, Shea Butter oder Illipé Butter, aus Kohlenwasserstoffen oder Mischungen hiervon;
wobei die Lipidkomponente mindestens ein flüssiges Öl umfasst;
20 bis 55 Gew. % eines oder mehrere Tenside, ausgewählt aus anionischen, amphoteren, nichtionischen Tensiden der Gruppe, umfassend C₁₀-C₁₈- Alkylsulfate, C₁₀-C₁₈- Alkylethersulfate, C₁₀-C₁₈- Alkylethersulfate mit einem Ethoxylierungsgrad von 2 bis 6, C₁₀-₁₈Alkyl-Sarcosinate, C₁₀-₁₈ ⁻Alkylsethionate, C₁₀-₁₈ Alkyl-EtherCarbonsäuren, Eiweiß-Fettsäure-Kondensate, C₁₀-₁₈ Monoalkylphosphate, C₁₀-₁₈⁻ Monoglyceridsulfate, C₁₀-₁₈ ⁻Alkyl-Amidethersulfaten, C₁₀-₁₈- Alkylsulfoacetaten;
C₈-C₂₂- Alkanolamide, C₁₀-C₁₈-Fettsäureethoxylate mit 8-40 EO Einheiten, C₁₀-C₁₀ Alkylpolyglucoside, Sorbitan(C₈-C₂₂)Alkylester; C₈-C₂₂⁻Alkylaminopropionate, C₈-C₂₂Alkyl-, C₈-C₂₂Alkylamido- und -Sulfo-Betaine, C₈-C₂₂-Alkylglycinate;
oder Mischungen hiervon;
0,1 bis 5 Gew.% eines oder mehrerer Vesikelbildenden Lipid-Substanz(en), ausgewählt aus Phospholipiden und Sphingolipiden;
sowie mehr als 8 bis 27 Gew.% eines oder mehrerer Wirkstoffe, ausgewählt aus ätherischen Ölen/Pflanzenextrakten oder Mischungen hiervon,

2. Zubereitung nach Anspruch 1, **dadurch gekennzeichnet, dass** sie 7 bis 60 Gew.% Wasser aufweist.

3. Zubereitung nach Anspruch 1 oder 2, **dadurch gekennzeichnet, dass** sie 10 bis 25 Gew. % eines oder mehrerer Wirkstoffe, ausgewählt aus ätherischen Ölen/Pflanzenextrakten oder Mischungen hiervon aufweist.

4. Zubereitung nach einem der Ansprüche 1 bis 3, **dadurch gekennzeichnet, dass** das oder die Lipide der Lipidkomponente ausgewählt sind aus Olivenöl, Sonnenblumen-, Sojaöl, Aprikosenkern-, Traubenkernöl, Haselnussöl, Mohnöl-, Erdnuß-, Mandel-, Weizenkeim-, Sesam-, Distelöl, Avocadoöl, Borretschsamenöl, Sanddornöl, Maiskeimöl, Walnussöl, Nachtkerzenöl, Palmöl, Pinienkernöl, Reiskeimöl, Kürbiskernöl, Marulaöl, oder Mischungen dieser Öle.

5. Zubereitung nach einem der Ansprüche 1 bis 4, **dadurch gekennzeichnet, dass** sie 0,1 bis 20 Gew.% Zusatzstoffe, ausgewählt aus Parfümstoffen, Konsistenzreglern (Konditioniermitteln), Konservierungsmitteln, Stabilisatoren, Farbstoffen; und/ oder 0,1 bis 15 Gew.% Zusatzwirkstoffe, ausgewählt aus Pflegestoffen, durchblutungs-fördernden Stoffen, hygienischen/dermatologisch-medizinischen Wirkstoffen, Vitaminen oder Mischungen hiervon aufweist.

6. Zubereitung gemäß Anspruch 5, **dadurch gekennzeichnet, dass** das oder die anionischen Tenside ausgewählt ist/sind aus C₁₀-C₁₈- Alkylethersulfaten; C₁₀-C₁₈- Alkylethersulfaten mit einem Ethoxylierungsgrad von 2 bis 6, C₁₀-₁₈ -Alkyl-Sarcosinaten oder Mischungen hiervon.

7. Zubereitung nach einem der Ansprüche 1 bis 6, **dadurch gekennzeichnet, dass** sie in 1 bis 6 Gew.% Lipid(e), 25 bis 49 Gew.% Tensid(e), 15 bis 23 Gew.% Wirkstoff(e), 0,3 bis 4 Gew. % vesikelbildende(s) Lipid(e), 0,1 bis 25 Gew.% Zusatzstoffe, 0,5 bis 15 Gew.% Zusatzwirkstoffe sowie als Rest, z. B. 20 bis 53 % Wasser aufweist.

8. Zubereitung nach einem der Ansprüche 1 bis 7, **dadurch gekennzeichnet, dass** sie 15 bis 19,3 Gew. % Wirkstoff(e) aufweist.

9. Zubereitung nach einem der Ansprüche 1 bis 8, **dadurch gekennzeichnet, dass** das oder die vesikelbildnende(n) Lipid(e) ausgewählt ist/sind aus Lecithinen, Phosphatidylcholin, oder -diethanolamin, Phosphatidylserin, Phosphatidylinosit, (z.B. aus Soja, Raps, Baumwollsamen, Ei), Sphingosin, Ceraminden, Sphingomyelin sowie deren Mischungen.

10. Zubereitung nach einem der Ansprüche 1 bis 9, **dadurch gekennzeichnet, dass** das oder die Lipide ausgewählt sind aus Mandelöl, Avocadoöl, Marulaöl, Aprikosenkernöl, Haselnussöl, Sanddornöl, Sojaöl oder Mischungen hiervon.

11. Zubereitung nach einem der Ansprüche 1 bis 10 zur kosmetischen Anwendung als Erkältungsbad, Rheumabad, Entspannungsbad, Erfrischungs-/Belebungsbad, Abhärtungsbad, Anti-Stress-, Muskel-Lockerungs-Bad, Durchblutungsbad, Hautpflegebad.

12. Kosmetische, nicht therapeutische Verwendung einer Zubereitung gemäß Anspruch 11 für Voll- oder Teilbäder, für die Wanne, Fußbad, Armbad.

## Claims

1. A liposome-forming bath preparation comprising:
water;
1 to 8 wt.% of one or more lipids as lipid component selected from liquid oils, selected from apricot kernel oil, avocado oil, borage seed oil, thistle oil, peanut oil, hazelnut oil, pumpkin seed oil, maize germ oil, almond oil, marula oil, poppy seed oil, evening primrose oil, olive oil, palm oil, pine nut oil, rice germ oil, sea buckthorn oil, sesame oil, soy oil, sunflower oil, grapeseed oil, walnut oil, wheat germ oil, from semisolid lipids of the group comprising jojoba oil or the substitute thereof oleyl erucate, shea butter or illipe butter, from hydrocarbons or mixtures thereof;
wherein the lipid component comprises at least one liquid oil;
20 to 55 wt.% of one or more surfactants selected from anionic, amphoteric, nonionic surfactants of the group comprising C₁₀-C₁₈ alkyl sulfates, C₁₀-C₁₈ alkyl ether sulfates, C₁₀-C₁₈ alkyl ether sulfates with a degree of ethoxylation of 2 to 6, C₁₀-₁₈ alkyl sarcosinates, C₁₀-₁₈ alkyl isethionates, C₁₀-₁₈ alkyl ether carboxylic acids, protein-fatty acid condensation products, C₁₀-₁₈ monoalkyl phosphates, C₁₀-₁₈ monoglyceride sulfates, C₁₀-₁₈ alkyl amide ether sulfates, C₁₀-₁₈ alkyl sulfoacetates; C₈-C₂₂ alkanolamides, C₁₀-C₁₈ fatty acid ethoxylates with 8-40 EO units, C₁₀-C₁₆ alkyl polyglucosides, sorbitan (C₈-C₂₂)-alkyl esters; C₈-C₂₂ alkylaminopropionates, C₈-C₂₂ alkyl-, C₈-C₂₂ alkylamido- and sulfo-betaines, C₈-C₂₂ alkyl glycinates;
or mixtures thereof;
0.1 to 5 wt.% of one or more vesicle-forming lipid substance(s) selected from phospholipids and sphingolipids;
together with more than 8 to 27 wt.% of one or more active ingredients, selected from essential oils/plant extracts or mixtures thereof.

2. A preparation according to claim 1, **characterised in that** it comprises 7 to 60 wt.% of water.

3. A preparation according to claim 1 or 2, **characterised in that** it comprises 10 to 25 wt.% of one or more active ingredients selected from essential oils/plant extracts or mixtures thereof.

4. A preparation according to one of claims 1 to 3, **characterised in that** the lipid(s) of the lipid component are selected from olive oil, sunflower oil, soy oil, apricot kernel oil, grapeseed oil, hazelnut oil, poppy seed oil, peanut oil, almond oil, wheat germ oil, sesame oil, thistle oil, avocado oil, borage seed oil, sea buckthorn oil, maize germ oil, walnut oil, evening primrose oil, palm oil, pine nut oil, rice germ oil, pumpkin seed oil, marula oil, or mixtures of these oils.

5. A preparation according to one of claims 1 to 4, **characterised in that** it comprises 0.1 to 20 wt.% of additives selected from perfume substances, consistency regulators (conditioners), preservatives, stabilisers, dyes; and/or 0.1 to 15 wt.% of additional active ingredients selected from conditioning substances, circulation-promoting substances, hygiene/dermatological medical active ingredients, vitamins or mixtures thereof.

6. A preparation according to claim 5, **characterised in that** the anionic surfactant(s) is/are selected from C₁₀-C₁₈ alkyl ether sulfates; C₁₀-C₁₈ alkyl ether sulfates with a degree of ethoxylation of 2 to 6, C₁₀-₁₈ alkyl sarcosinates or mixtures thereof.

7. A preparation according to one of claims 1 to 6, **characterised in that** it comprises 1 to 6 wt.% of lipid(s), 25 to 49 wt.% of surfactant(s), 15 to 23 wt.% of active ingredient(s), 0.3 to 4 wt.% of vesicle-forming lipid(s), 0.1 to 25 wt.% of additives, 0.5 to 15 wt.% of additional active ingredients with the remainder, for example 20 to 53%, being water.

8. A preparation according to one of claims 1 to 7, **characterised in that** it comprises 15 to 19.3 wt.% of active ingredient(s).

9. A preparation according to one of claims 1 to 8, **characterised in that** the vesicle-forming lipid(s) is/are selected from lecithins, phosphatidylcholine or phosphatidyldiethanolamine, phosphatidylserine, phosphatidylinositol, (for example from soy, oilseed rape, cotton seeds or egg), sphingosine, ceramides, sphingomyelin and mixtures thereof.

10. A preparation according to one of claims 1 to 9, **characterised in that** the lipid(s) is/are selected from almond oil, avocado oil, marula oil, apricot kernel oil, hazelnut oil, sea buckthorn oil, soya oil or mixtures thereof.

11. A preparation according to one of claims 1 to 10 for cosmetic use as a common cold bath, rheumatic bath, relaxation bath, refreshing/vitalising bath, resistance-developing bath, stress-relieving/muscle-relaxing bath, circulatory bath or skin-care bath.

12. Cosmetic, non-therapeutic use of a preparation according to claim 11 for baths for the whole body or body parts for a bathtub, foot bath or arm bath.

## Revendications

1. Préparation pour le bain formant des liposomes, comprenant :
de l'eau ;
1 à 8% en poids d'un ou de plusieurs lipides comme composant lipidique, choisis parmi les huiles liquides, choisies parmi l'huile de noyau d'abricot, l'huile d'avocat, l'huile de graines de bourrache, l'huile de carthame, l'huile d'arachide, l'huile de noisette, l'huile de pépins de courge, l'huile de germe de maïs, l'huile d'amande, l'huile de marula, l'huile d'oeillette, l'huile d'onagre, l'huile d'olive, l'huile de palme, l'huile de pignon, l'huile de germe de riz, l'huile d'argousier, l'huile de sésame, l'huile de soja, l'huile de tournesol, l'huile de pépins de raisin, l'huile de noix, l'huile de germe de blé, parmi les lipides semi-solides du groupe comprenant l'huile de jojoba ou son substitut érucate d'oléyle, le beurre de karité ou le beurre d'illipé, parmi les hydrocarbures ou les mélanges de ceux-ci ; le composant lipidique comprenant au moins une huile liquide ;
20 à 55% en poids d'un ou de plusieurs tensioactifs, choisis parmi les tensioactifs anioniques, amphotères, non ioniques du groupe comprenant les sulfates de C₁₀-C₁₈-alkyle, les C₁₀-C₁₈-alkyléthersulfates, les C₁₀-C₁₈-alkyléthersulfates présentant un degré d'éthoxylation de 2 à 6, les sarcosinates de C₁₀-₁₈-alkyle, les iséthionates de C₁₀-₁₈-alkyle, les acides C₁₀-₁₈-alkyléthercarboxyliques, les produits de condensation de protéines-acides gras, les monophosphates de C₁₀-₁₈-alkyle, les monosulfates de C₁₀-₁₈-monoglycérol, les amidoéthersulfates de C₁₀-₁₈-alkyle, les sulfoacétates de C₁₀-₁₈-alkyle ; les C₈-C₂₂-alcanolamides, les éthoxylates d'acides gras en C₁₀-C₁₈ comprenant 8-40 unités d'OE, les C₁₀-C₁₆-alkylpolyglucosides, les (C₈-C₂₂)-alkylesters de sorbitane ; les aminopropionates de C₈-₂₂-alkyle, les C₈-C₂₂-alkylbétaïnes, les C₈-C₂₂-alkylamidobétaïnes et les C₈-C₂₂-sulfobétaïnes, les glycinates de C₈-C₂₂-alkyle ; ou les mélanges de ceux-ci ;
0,1 à 5% en poids d'une ou de plusieurs substance(s) lipidique(s) formant des vésicules, choisies parmi les phospholipides et les sphingolipides ;
ainsi que plus de 8 jusqu'à 27% en poids d'une ou de plusieurs substances actives, choisies parmi les huiles essentielles/extraits végétaux ou les mélanges de ceux-ci.

2. Préparation selon la revendication 1, **caractérisée en ce qu'**elle présente 7 à 60% en poids d'eau.

3. Préparation selon la revendication 1 ou 2, **caractérisée en ce qu'**elle présente 10 à 25% en poids d'une ou de plusieurs substances actives, choisies parmi les huiles essentielles/extraits végétaux ou les mélanges de ceux-ci.

4. Préparation selon l'une quelconque des revendications 1 à 3, **caractérisée en ce que** le ou les lipides du composant lipidique sont choisis parmi l'huile d'olive, l'huile de tournesol, l'huile de soja, l'huile de noyau d'abricot, l'huile de pépins de raisin, l'huile de noisette, l'huile d'oeillette, l'huile l'arachide, l'huile d'amande, l'huile de germe de blé, l'huile de sésame, l'huile de carthame, l'huile d'avocat, l'huile de graines de bourrache, l'huile d'argousier, l'huile de germe de maïs, l'huile de noix, l'huile d'onagre, l'huile de palme, l'huile de pignon, l'huile de germe de riz, l'huile de pépins de courge, l'huile de marula ou les mélanges de ces huiles.

5. Préparation selon l'une quelconque des revendications 1 à 4, **caractérisée en ce qu'**elle contient 0,1 à 20% en poids d'additifs, choisis parmi les substances parfumées, les agents de régulation de la consistance (agents de conditionnement), les conservateurs, les stabilisants, les colorants ; et/ou 0,1 à 15% en poids de substances actives auxiliaires, choisies parmi les agents de soins, les substances favorisant la circulation sanguine, les substances actives hygiéniques/dermatologiques-médicales, les vitamines ou les mélanges de ceux-ci.

6. Préparation selon la revendication 5, **caractérisée en ce que** le ou les tensioactifs anioniques est/sont choisi(s) parmi les C₁₀-C₁₈-alkyléthersulfates ; les C₁₀-C₁₈-alkyléthersulfates présentant un degré d'éthoxylation de 2 à 6, les sarcosinates de C₁₀-C₁₈-alkyle ou les mélanges de ceux-ci.

7. Préparation selon l'une quelconque des revendications 1 à 6, **caractérisée en ce qu'**elle présente 1 à 6% en poids de lipide(s), 25 à 49% en poids de tensioactif(s), 15 à 23% en poids de substance(s) active(s), 0,3 à 4% en poids de lipide(s) formant des vésicules, 0,1 à 25% en poids d'additifs, 0,5 à 15% en poids de substances actives auxiliaires ainsi que comme reste par exemple 20 à 53% d'eau.

8. Préparation selon l'une quelconque des revendications 1 à 7, **caractérisée en ce qu'**elle présente 15 à 19,3% en poids de substance(s) active(s).

9. Préparation selon l'une quelconque des revendications 1 à 8, **caractérisée en ce que** le ou les lipide(s) formant des vésicules est/sont choisi(s) parmi les lécithines, la phosphatidylcholine ou la phosphatidyldiéthanolamine, la phosphatidylsérine, le phosphatidylinositol, (par exemple de soja, de colza, de graines de coton, d'oeuf), la sphingosine, les céramides, la sphingomyéline ainsi que leurs mélanges.

10. Préparation selon l'une quelconque des revendications 1 à 9, **caractérisée en ce que** le ou les lipides sont choisis parmi l'huile d'amande, l'huile d'avocat, l'huile de marula, l'huile de noyau d'abricot, l'huile de noisette, l'huile d'argousier, l'huile de soja ou les mélanges de celles-ci.

11. Préparation selon l'une quelconque des revendications 1 à 10 pour l'utilisation cosmétique comme bain contre le refroidissement, bain contre les rhumatismes, bain de détente, bain de rafraîchissement/stimulation, bain d'endurcissement, bain antistress, bain de relâchement musculaire, bain pour la circulation sanguine, bain de soin pour la peau.

12. Utilisation cosmétique, non thérapeutique d'une préparation selon la revendication 11 pour des bains complets ou partiels, pour la baignoire, le bain de pieds, le bain pour les bras.
